(19) Europäisches Patentamt
European Patent Office
Office européen des brevets

(11) Veröffentlichungsnummer : **0 326 875 B1**

(12) # EUROPÄISCHE PATENTSCHRIFT

(45) Veröffentlichungstag der Patentschrift : **25.03.92 Patentblatt 92/13**

(51) Int. Cl.$^5$ : **C07C 401/00,** A61K 31/59

(21) Anmeldenummer : **89100973.0**

(22) Anmeldetag : **20.01.89**

(54) **Didehydro-vitamin D3-Derivate.**

(30) Priorität : **20.01.88 US 145867**

(43) Veröffentlichungstag der Anmeldung : **09.08.89 Patentblatt 89/32**

(45) Bekanntmachung des Hinweises auf die Patenterteilung : **25.03.92 Patentblatt 92/13**

(84) Benannte Vertragsstaaten : **AT BE CH DE ES FR GB GR IT LI LU NL SE**

(56) Entgegenhaltungen :
**EP-A- 0 122 490**
**WO-A-85/03298**
**DE-A- 2 812 741**
**DE-A- 2 920 092**
**DE-A- 3 243 073**
**DE-A- 3 541 934**

(73) Patentinhaber : **F. HOFFMANN-LA ROCHE AG**
**Postfach 3255**
**CH-4002 Basel (CH)**

(72) Erfinder : **Baggiolini, Enrico Giuseppe**
**30 Evergreen Drive**
**North Caldwell, N.J. 07006 (US)**
Erfinder : **Partridge, John Joseph**
**620 Airport Road**
**Chapel Hill North Carolina 27514 (US)**
Erfinder : **Shiuey, Shian-Jan**
**331 Bloomfield Avenue**
**Nutley, N.J. 07110 (US)**
Erfinder : **Truitt, Gary Arthur**
**109 Garner Avenue**
**Bloomfield, N.J. 07003 (US)**
Erfinder : **Uskokovic, Milan Radoje**
**253 Highland Avenue**
**Upper Montclair, N.J. 07043 (US)**

(74) Vertreter : **Mahé, Jean et al**
**Postfach 3255 Grenzacherstrasse 124**
**CH-4002 Basel (CH)**

EP 0 326 875 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Verbindungen der Formel

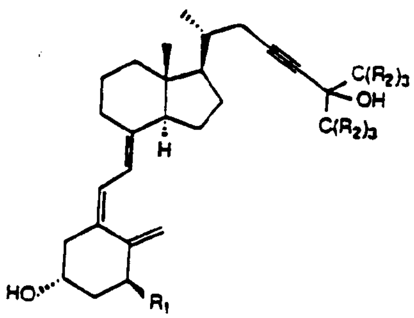

I

worin $R_1$ Wasserstoff oder Hydroxy und $R_2$ Wasserstoff oder Fluor ist,
pharmazeutische Präparate enthaltend eine Verbindung der Formel I und die Verwendung dieser Verbindungen bei der Herstellung von solchen Präparaten, verwendbar in der Behandlung von Krankheiten, die durch Mangelzustände des Kalzium-Metabolismus charakterisiert sind, insbesondere der Osteoporose und der renalen Osteodystrophie. Ferner betrifft die Erfindung ein Verfahren zur Herstellung eines Arzneimittels, wobei eine Verbindung der Formel I evtl. zusammen mit einem pharmazeutisch geeigneten Trägermaterial und sonstigen Hilfsstoffen in eine für therapeutische Zwecke anwendbare Form gebracht wird, dadurch gekennzeichnet, dass man ein Heilmittel für die Behandlung der besagten Krankheiten herstellt, bestehend aus der Kombination eines eine Verbindung der Formel I enthaltenden Präparats zusammen mit der Information, welche in direktem Zusammenhang mit der Behandlung dieser Krankheiten steht.

Beispiele von $C_{1-8}$-Alkylgruppen sind Methyl, Aethyl, Propyl, Isopropyl, t-butyl, Hexyl, Heptyl und Octyl. Beispiele von Arylgruppen sind unsubstituiertes Phenyl und Phenyl substituiert durch $C_{1-8}$-Alkyl, Fluor, Chlor, Brom, Jod, Nitro, Cyan oder Trifluormethyl. Beispiele von Hydroxyschutzgruppen sind -(CO)-$C_{1-8}$-Alkyl und Tri-($C_{1-8}$-alkyl)silyl.

Bevorzugte Verbindungen der Formel I sind:

1α,25-Dihydroxy-23,24-didehydrocholecalciferol;
25-Hydroxy-23,24-didehydrocholecalciferol;
1α,25-Dihydroxy-23,24-didehydro-26,26,26,27,27,27-hexafluorcholecalciferol; und
25-Hydroxy-23,24-didehydro-26,26,26,27,27,27-hexafluorcholecalciferol,

nachstehend als Verbindungen A, B, C bzw. D bezeichnet.

Die Verbindungen der Formel I, d.h. diejenigen der Formeln Ia und Ib, kann man wie in den nachstehenden Schemata 1, 2 und 3 beschrieben, herstellen.

Schema 1

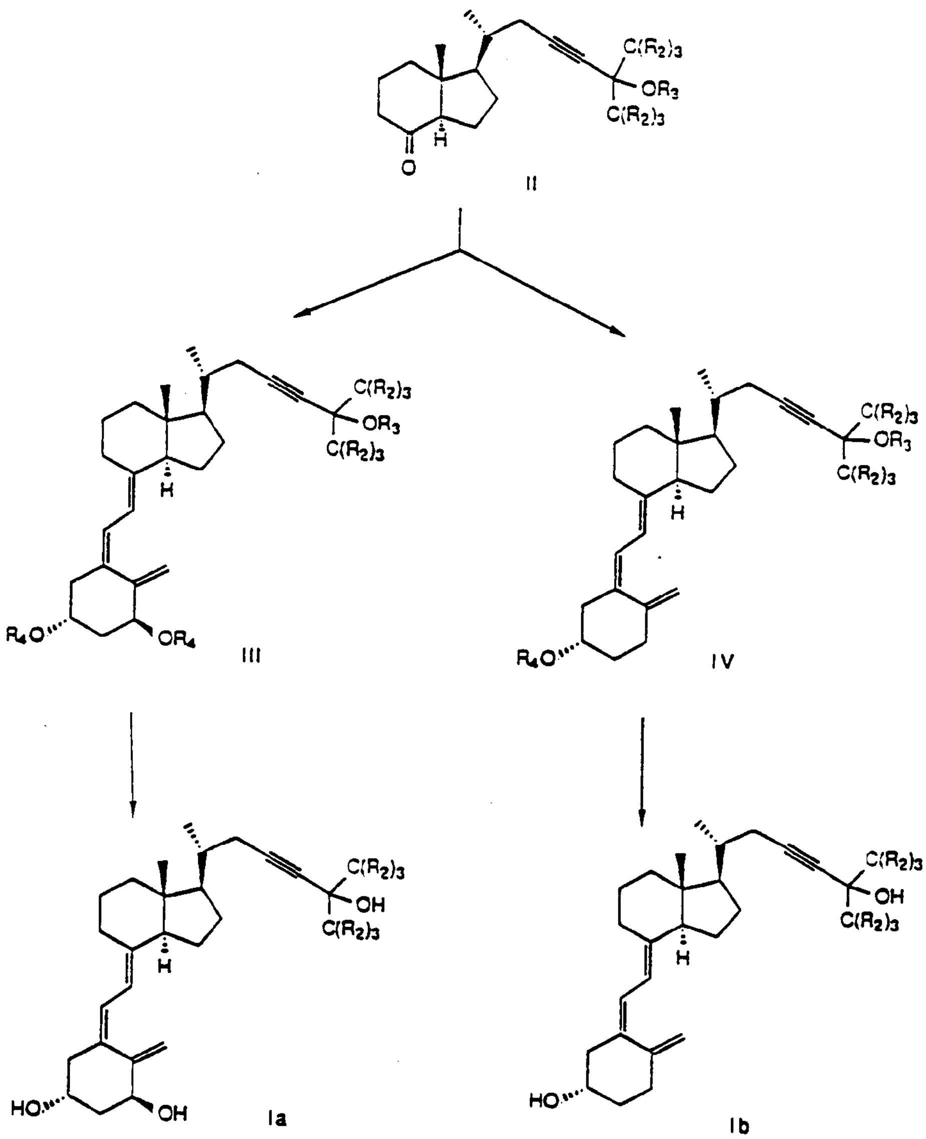

II

III

IV

Ia

Ib

worin $R_2$ die obige Bedeutung hat, $R_3$ und $R_4$ $-Si(R_5)_3$, $R_5$ $C_{1-8}$-Alkyl, Aryl oder Aryl-$C_{1-8}$-alkyl darstellen.

Schema 2

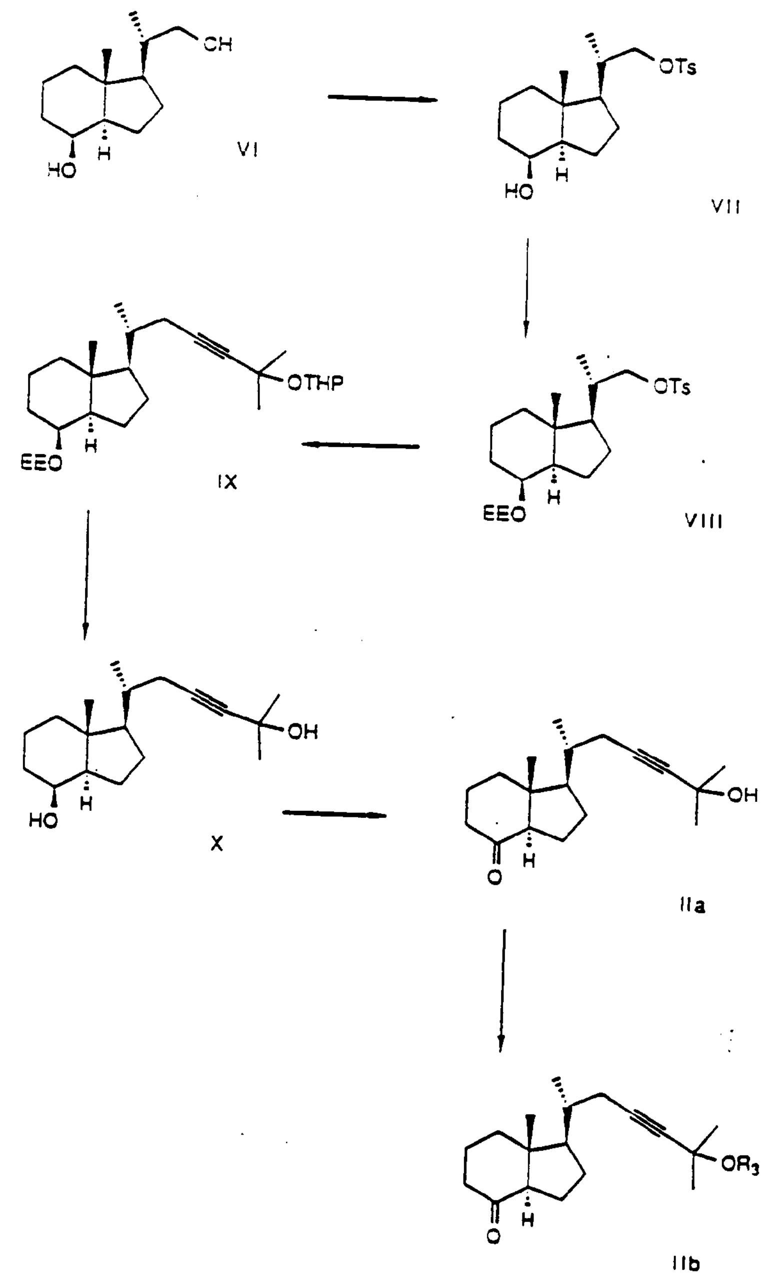

VI

VII

VIII

IX

X

IIa

IIb

worin Ts Tosyl, OTHP Tetrahydropyranyloxy, EEO Aethoxyäthoxy ist und $R_3$ die obige Bedeutung hat.

Schema 3

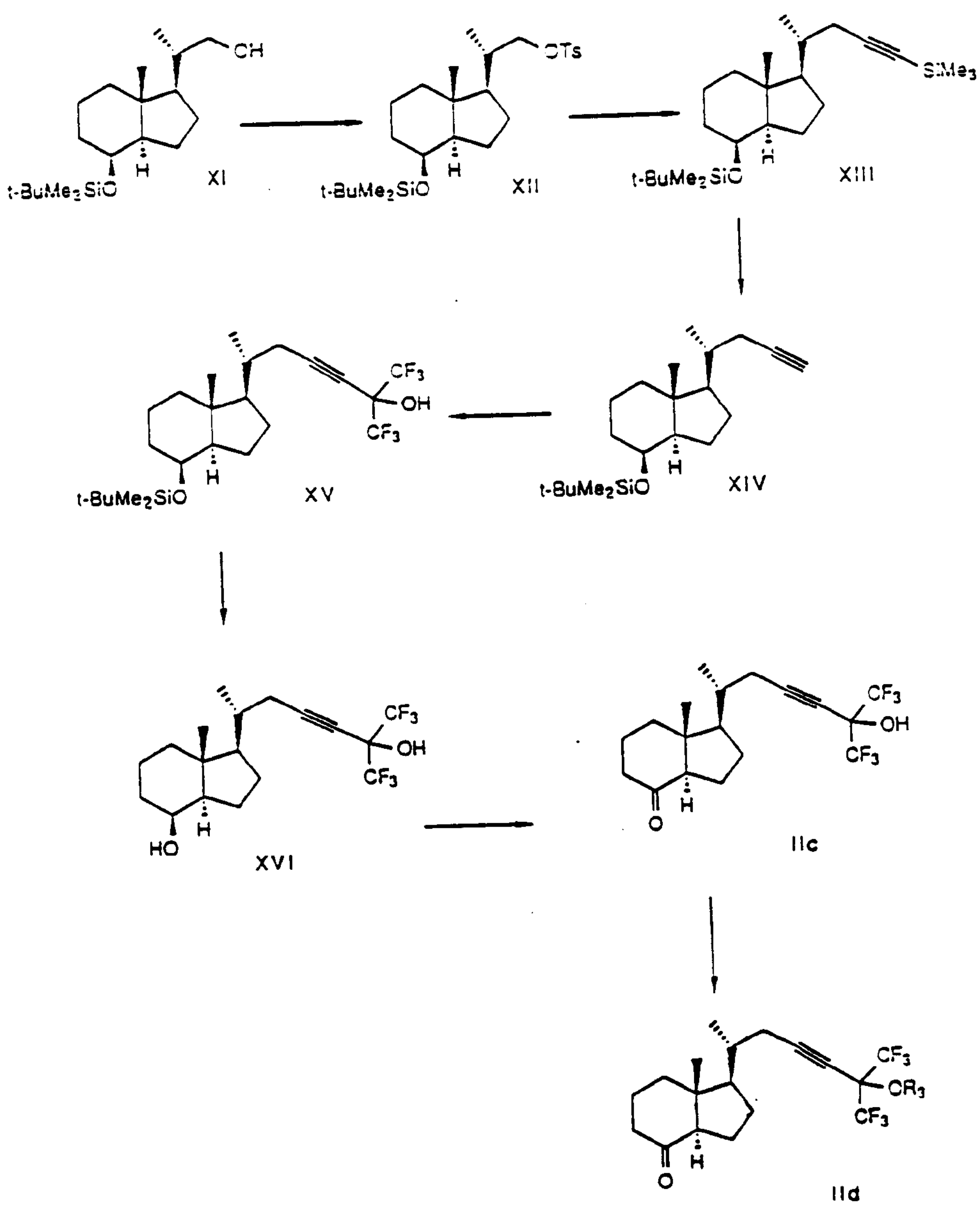

XI XII XIII XIV XV XVI IIc IId

worin $R_3$ die obige Bedeutung hat.

Eine Verbindung der Formel II kann mit einer Verbindung der Formel

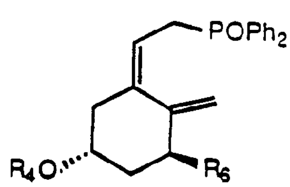

V

worin $R_6$ Wasserstoff oder $OR_4$ ist, und $R_4$ die obige Bedeutung hat, zu einer Verbindung der Formel III oder

IV umgesetzt werden.

Die Verbindungen der Formel V sind bekannt oder können in an sich bekannter Weise hergestellt werden, z.B. wie beschrieben in J. Org. Chem. 51, 1986, 3098. Die Reaktion wird in Gegenwart einer starken Base, z.B. einem Alkyllithium-Derivat oder einem mono- oder dialkylierten Disilylamid in einem herkömmlichen Aether unter einer inerten Atmosphäre und bei einer Temperatur im Bereich von etwa -80 bis -50°C.

Die Verbindungen der Formel III oder IV werden in die entsprechenden Cholecalciferolderivate der Formel Ia oder Ib übergeführt durch Entfernung der Hydroxyschutzgruppen, vorzugsweise durch Behandlung mit einem organischen Fluorid, z.B. Tetrabutylammoniumfluorid, bei Raumtemperatur und in einem Lösungsmittel, z.B. Tetrahydrofuran (THF). Alternativ kann die Abspaltung der Hydroxyschutzgruppen durch Behandlung einer Verbindung der Formel III oder IV mit einem $C_{1-8}$-Alkanol oder mit einem Gemisch von Wasser und einem mischbaren organischen Lösungsmittel in Gegenwart einer Säure, z.B. einer anorganischen Säure, einer Niederalkancarbonsäure oder einer Sulfonsäure, vorzugsweise mit einem kationischen Austauscherharz in H-Form, wie AG50W-X4 Bio-Rad Laboratories, Amberlite CG120, Amerlyst 15 oder Dowex 50X4, als Suspension in einem $C_{1-8}$-Alkanol, durchgeführt werden.

Eine Verbindung der Formel IIa, d.h. der Formel II, worin $R_2$ und $R_3$ Wasserstoff sind, kann durch Tosylierung der Verbindung der Formel VI (Tetrahedron 40, 1984, 2283) zur Verbindung der Formel VII, z.B. mit p-Toluolsulfonylchlorid, in einem basischen Lösungsmittel, wie Collidin oder Pyridin, bei etwa -10 bis 10°C, vorzugsweise bei 0°C, unter einer inerten Atmosphäre, wie Stickstoff, hergestellt werden.Die Verbindung der Formel VII wird dann in eine solche der Formel VIII übergeführt, durch Reaktion mit Aethylvinyläther in einem aprotischen Lösungsmittel und in Gegenwart einer Säure, z.B. Benzoesäure oder p-Toluolsulfonsäure, bei etwa -90 bis -60°C, vorzugsweise bei -70°C, unter einer inerten Atmosphäre wie Stickstoff.

Die Verbindung der Formel VIII wird in eine solche der Formel IX übergeführt durch Rühren mit einem Lithiumderivat des Tetrahydropyranyläthers des 3-Methyl-1-butyn-3-ols, vorzugsweise mittels eines Alkyllithiums, wie n-Butyllithium, und trockenem Dioxan, bei einer Temperatur von 0 bis 5°C und Erhitzen bis zur Rückflusstemperatur, wobei alle diese Reaktionen unter einer inerten Atmosphäre, z.B. Argon, durchgeführt werden. Die Verbindung der Formel IX wird dann in eine solche der Formel X übergeführt durch Reaktion mit einer Säure, z.B. p-Toluolsulfonsäure in einem Niederalkanol, z.B. Methanol, zunächst bei etwa -10 bis 10°C, vorzugsweise bei 0°C, und dann bei etwa Raumtemperatur.

Die Verbindung der Formel X wird zu einer Verbindung der Formel IIa oxidiert, z.B. mittels Pyridiniumchlorochromat in einem Lösungsmittel, wie einem $C_{1-8}$-Alkylhalogenid, z.B. Chloroform, Tetrachlorkohlenstoff oder Dichloromethan, bei etwa -10 bis 30°C, vorzugsweise bei Raumtemperatur. Das Keton der Formel IIa kann zu einem solchen der Formel IIb umgewandelt werden durch Behandlung mit einem Silylierungsmittel, wie einem tri($R_5$)-substituierten Silylimidazol, worin $R_5$ die obige Bedeutung hat, z.B. mit Trimethylsilylimidazol, in einem inerten Lösungsmittel, wie einem Aether oder einem halogenierten Kohlenwasserstoff, wie Dichlormethan, unter einer inerten Atmosphäre, wie Argon.

Eine Verbindung der Formel IIc, d.h. der Formel II, worin $R_2$ Fluor und $R_3$ Wasserstoff ist, kann hergestellt werden durch Tosylierung der Verbindung der Formel XI zur Verbindung der Formel XII in analoger Weise wie oben beschrieben für die Ueberführung einer Verbindung der Formel VI zu einem Tosylat der Formel VII.

Die Verbindung der Formel XIII kann man herstellen durch Reaktion des n-Butyllithiumderivates von (Trimethylsilyl)acetylen mit der Verbindung der Formel XII und Erhitzen des entstandenen Gemisches bei Rückflusstemperatur. Die Verbindung der Formel XIII wird in eine solche der Formel XIV übergeführt durch Reaktion mit Silbernitrat und dann mit Kaliumcyanid in einem wässrigen Alkanol, z.B. in Aethanol und Wasser.

Die Verbindung der Formel XIV wird in eine solche der Formel XV übergeführt durch Reaktion mit gasförmigem Hexafluoraceton in Gegenwart eines Alkyllithiums, z.B. Butyllithium, in einem Aether, z.B. THF, bei etwa -90 bis -70°C, vorzugsweise bei -75°C. Die Verbindung der Formel XV wird in eine solche der Formel XVI übergeführt durch Reaktion mit Fluorwasserstoffsäure in Azetonitril und THF. Die Verbindung der Formel XVI wird oxidiert zu einer solchen der Formel IIc durch Reaktion mit einem Oxidierungsmittel in einem $C_{1-8}$-Alkylhalogenid (wie weiter oben beschrieben für die Ueberführung eines Alkohols X in das Keton IIa) in Gegenwart von wasserfreiem Natriumacetat. Die Verbindung der Formel IIc kann in eine solche der Formel IId übergeführt werden, mittels eines Silylierungsmittels wie weiter oben beschrieben für die Umwandlung von IIa in IIb.

Die Stimulierung der intestinalen Kalziumabsorption (ICA) und der Knochenkalziummobilisierung (BCM) an der Ratte, sowie die kompetitive Bindung (CB) an intestinalen Rezeptoren des 1$\alpha$,25-Dihydroxycholecalciferols (nachstehend Verbindung X oder 1,25$(OH)_2D_3$) wurden an der Ratte, dem Küken und dem Kalb gemessen. In der nachstehenden Tabelle I sind die Resultate für die weiter oben definierten Verbindungen A, B, C und D angegeben in Prozenten der Werte von 1,25$(OH)_2$-$D_3$:

Tabelle I

| | Effekt in der Ratte als % von $1,25(OH)_2D_3$ | | | Kompetive Bindung (CB) an intestinalen Rezeptoren, als % des CB von $1,25(OH)_2D_3$ | | |
|---|---|---|---|---|---|---|
| Verbindung | Konzentration (mg/Ratte) | ICA | BCM | Ratte | Küken | Kalb |
| X | 12.5 | 100 | 100 | 100 | 100 | 100 |
| A | 15.5 | 95 | 15 | 39 | 60 | 47 |
| B | 12.5 | 71 | 0 | | 0 | 0 |
| C | 12.5 | 124 | 0 | 142 | 47 | 62 |
| D | 12.5 | 0 | 11 | | 0 | 0 |

Diese Resultate zeigen einerseits dass im Vergleich zu $1,25(OH)_2D_3$, bei den Verbindungen A bis D die intestinale Kalziumabsorption höher ist als die Knochenkalziummobilisierung und ferner dass die Verbindungen A bis D an den intestinalen $1,25(OH)_2D_3$-Rezeptoren binden.

Die Konzentration der Kalzium- und Phosphationen und diejenige von Kreatinin im Serum, sowie das Körper- und Knochengewicht wurden sowohl bei Vitamin $D_3$-defizienten Ratten, die mit $1,25(OH)_2D_3$ oder mit der Verbindung C behandelt wurden, gemessen, wie auch bei Ratten, die mit Vitamin $D_3$-reichem Futter (D+-Kontrolle) oder mit Vitamin $D_3$-armem Futter (D--Kontrolle) behandelt wurden. Die Resultate sind in der Tabelle II angegeben:

Tabelle II

| | Tägliche Dosis mg | Körpergewicht g | $Ca^{2+}$ mg/dl | $PO_4^{2-}$ | Kreatinin mg/dl | Knochengewicht $mg/cm^3$ |
|---|---|---|---|---|---|---|
| $1,25-(OH)_2D_3$ | 15 | 192±4.5 | 11.76±0.16 | 7.61±0.31 | 0.37±0.04 | 156±16 |
| | 60 | 156±15 | 11.92±0.40 | 6.58±0.30 | 0.36±0.02 | 296±56 |
| Verbindung C | 18 | 148±4.3 | 15.63±0.36 | 7.66±0.17 | 0.60±0.05 | 385±17 |
| | 72 | 111±5.9 | 15.23±0.22 | 6.34±0.26 | 0.43±0.06 | 746±48 |
| D+ Kontrolle | | 148±1.3 | 5.60±0.5 | 8.65±0.6 | 0.44±0.04 | 96.5±9 |
| D- Kontrolle | | 202±3.1 | 10.41±0.10 | 5.76±0.12 | 0.41±0.01 | 168±15 |

Die antiproliferativen (AP) und die Differenzierung induzierenden (DI) Effekte der Verbindungen A bis D auf menschlichen promyelocytischen HL-60 Tumorzellen wurden evaluiert. In der Tabelle III ist der AP-Effekt in Prozent Reduktion der Anzahl Zellen und in Konzentration $ID_{50}$ dieser Verbindungen, die eine Reduktion der Anzahl Zellen um 50 % bewirkt, angegeben. Der DI-Effekt ist angegeben als % der differenzierten Zellen und als Konzentration $ED_{50}$ dieser Verbindungen, die zu einer 50 % Differenzierung der Zellen führt:

Tabelle III

| Verbindung | Konz. $x10^{-8}M$ | % Reduktion der Anzahl Zellen | $ID_{50}$ $x10^{-8}M$ | Differenzierte Zellen % | $ED_{50}$ $x10^{-8}M$ |
|---|---|---|---|---|---|
| A | 0.1 | 30 | | 15 | |
| | 1 | 67 | 0.6 | 54 | 0.9 |
| | 10 | 85 | | 98 | |
| B | 10 | 0 | | 5 | |
| | 100 | 31 | 150 | 27 | 150 |
| | 300 | 79 | | 95 | |
| C | 0.01 | 15 | | 13 | |
| | 0.1 | 31 | | 29 | |
| | 1 | 85 | 0.2 | 95 | 0.2 |
| | 10 | 89 | | 98 | |
| D | 1 | 0 | | 6 | |
| | 10 | 14 | 25 | 27 | 20 |
| | 100 | 77 | | 99 | |

Diese Daten zeigen, dass die Verbindungen der Formel I die Zellproliferation hemmen und die Zelldifferenzierung induzieren, und dass daher die besagten Verbindungen verwendbar sind als Mittel zur Behandlung von neoplastischen Krankheiten, wie Leukämie.

Die Verbindungen der Formel I können in Dosierungen im Bereich von etwa 0,1 oder 0,25 bis 2 mg pro Tag an Warmblüter abgereicht werden für die Behandlung von Krankheiten, die durch Mangelzustände des Kalziummetabolismus charakterisiert sind, wie renale Osteodystrophie und insbesondere Osteoporose. Beispiele von Präparaten enthaltend eine Verbindung der Formel I sind Tabletten, Kapseln und Elixire für die orale Verabreichung, oder sterile Lösung oder Suspensionen für die parenterale, z.B. subkutane, intramuskuläre, intravenöse oder intraperitoneale, Verabreichung. Die besagten Verbindungen können auch zur Herstellung von topischen Präparaten verwendet werden. Etwa 0,1 oder 0.25 bis etwa 2 mg einer Verbindung der Formel I kann mit einem pharmazeutisch verwendbaren Vehikel,
Träger, Excipienz, Bindemittel, Konservierungsmittel, Stabilisierungsmittel oder Riechstoff in einer Einzeldosisform verwendet werden.

Beispiele von Hilfsmitteln, die in Kapseln verwendet werden können, sind Bindemittel, wie Tragakant, Gummi oder Gelatine; Excipientien, wie Dikalziumphosphat; Sprengmittel, wie Maisstärke; Gleitmittel, wie Magnesiumstearat; Süssmittel, wie Sucrose oder Saccharin; Riechstoffe, wie Pfefferminz. Tabletten können mit Shellack, Zucker oder beides umhüllt sein. Ein Sirup oder ein Elixir kann ein Süssmittel, Methyl- und Propylparaben als Konservierungsmittel, einen Farbstoff und einen Riechstoff enthalten.

Sterile Präparate für Injektionen können dadurch hergestellt werden, dass man den Wirkstoff in einem Vehikel, wie Wasser, einem in der Natur vorkommenden pflanzlichen Oel, wie Sesamöl, oder einem synthetischen fetten Vehikel, wie Aethyloleat auflöst oder suspendiert. Man kann auch Puffer, Konservierungsmittel und Antioxidantien hinzufügen.

In den folgenden Beispielen sind die Temperaturen in Grad Celsius angegeben.

Beispiel 1

a) Ein Gemisch von 2,12 g (0,010 Mol) [1R-[1α,3aβ,4α,7aα]]-Octhahydro-4-hydroxy-β,7a-dimethyl-1H-inden-1-äthanol (J. Org. Chem., 48, 1983, 1414), 2.10 g p-Toluolsulfonylchlorid und 9 ml Pyridin wird 3 Stunden unter Stickstoff bei 0° gerührt. Das Gemisch wird in Eiswasser geschüttet und mit Methylenchlorid extrahiert. Die organische Schicht wird nacheinander mit Wasser, 1N Schwefelsäure und gesättigtem wässrigem Natriumbicarbonat gewaschen. Die Lösung wird getrocknet, filtriert und zur Trockene eingedampft. Man erhält 3,70 g [1R-[1α,3aβ,4α,7aα]]-Octahydro-4-hydroxy-β,7a-dimethyl -1H-inden-1-äthanol-α-(4-methylbenzolsulfonat), Smp. 97-98° nach Umkristallisation aus Methanol.

b) Ein Gemisch von 3,68 g (0,010 Mole) des Produkts von a), 100 ml Aethylvinylether und 0,04 g p-Toluolsulfonsäuremonohydrat wird 1 Stunde unter Stickstoff bei -70° gerührt und dann 1/2 Stunde auf 0° erwärmen gelassen. Das Gemisch wird mit 2 ml Triäthylamin behandelt und dann eingedampft. Der Rückstand wird in Methylenchlorid gelöst und mit gesättigter Bicarbonatlösung gewaschen. Die organische Phase wird getrocknet, filtriert und eingedampft. Man erhält 4.60 g [1R-[1α,3aβ,4α,7aα]]-4-(1-Aethoxyäthoxy)octahydro-β,7a-dimethyl-1H-inden-1-äthanol-4-methylbenzolsulfonat, $[\alpha]^{25}_D$+31° (c 1,2, $CHCl_3$).

c) Ein Gemisch von 1,26 g des Tetrahydropyranyläthers von 3-Methyl-1-butyn-3-ol und 5,0 ml 1,5M-Butyllithium in Hexan und 30 ml Dioxan wird 1/2 Stunde bei 5° und dann 1 Stunde bei Raumtemperatur unter Argon gerührt. Dann werden 1,32 g (0,0030 Mole) des Produkts von b) hinzugefügt und das Gemisch wird 36 Stunden bei Rückflusstemperatur erhitzt und abgekühlt. Das Gemisch wird in Wasser geschüttet und mit Aethylacetat extrahiert. Die organische Schicht wird mit Wasser und einer Salzlösung gewaschen und über Magnesiumsulfat getrocknet. Das Gemisch wird filtriert und eingedampft. Der Rückstand wird über Silicagel mit 4:1 Hexan-Aethylacetat gereinigt. Man erhält 1.43 g [1R-[1α,3aβ,4α,7aα]]-2[[5-4-Aethoxyäthoxy)-octahydro-7a-methyl1H-inden-1-yl]-1,1,5- trimethyl-2-pentynyl]oxy]tetrahydro-2Hpyran, $[\alpha]^{25}_D$+36° (c 1.0, $CHCl_3$).

d) Ein Gemisch von 3,50 g (0,0073 Mol) des Produkts von c), 50 ml Methanol und 0,10 g p-Toluolsulfonsäuremonohydrat wird 1/2 Stunde bei 0°C und 18 Stunden bei 23°C unter Stickstoff gerührt. Das Gemisch wird auf 10 ml konzentriert, dann mit Methylenchlorid verdünnt und mit gesättigtem wässrigem Natriumbicarbonat gewaschen. Die vereinigten wässrigen Phasen werden mit Methylenchlorid extrahiert. Die vereinigten organischen Phasen werden getrocknet, filtriert und eingedampft. Das Produkt wird über Silicagel mit 5:1 Hexan-Aethylacetat gereinigt. Man Man erhält 1,84 g
[1R-[1α,3aβ,4α,7aα]]-Octahydro-1-(5-hydroxy-1,5,5-trimethyl-3-pentynyl)-7a-methyl-1H-inden-4-ol, Smp. 62-63° nach Umkristallisation aus Ether-Hexan.

e) Einer Suspension von 2,40 g Pyridiniumchlorochromat in 50 ml Methylenchlorid werden bei 0°C 0,60 g des Produkt von d) in 10 ml Methylenchlorid zugesetzt. Das Gemisch wird 1/2 Stunde bei 0°C und 1 Stunde bei 23°C unter Stickstoff gerührt. Das Gemisch wird mit Aether verdünnt, 10 Minuten gerührt und dann filtriert. Der Filter wird mit Aether gewaschen und die vereinigten Filtrate werden eingedampft. Das entstandene Oel

wird in Aether suspendiert und filtriert, der Filter wird mit Aether gewaschen und die vereinigten Filtrate werden eingedampft. Das entstandene Oel wird über Silicagel mit 6:1 Hexan-Aethylacetat gereinigt. Man erhält 0,41 g [1R-[1α,3aβ,7aα]]-Octahydro-1-(5-hydroxy-1,5,5-trimethyl-3-pentynyl)-7a-methyl-4H- inden-4-on.

f) Ein Gemisch von 0,18 g (0,00065 Mol) des Produkts von e), 1,80 g Trimethylsilylimidazol und 5 ml Methylenchlorid wird 18 Stunden unter Argon bei 25° gerührt. Der Lösung wird 1 g Eis zugesetzt und das Gemisch wird 10 Minuten gerührt. Das Gemisch wird dann in Eiswasser geschüttet und mit Methylenchlorid extrahiert. Die vereinigten organischen Phasen werden mit Wasser gewaschen, getrocknet, filtriert und eingedampft. Das Produkt wird über Silicagel mit 6:1 Hexan-Aethylacetat gereinigt. Man erhält 0,21 g [1R-[1α,3aβ,7aα]]-Octahydro-1-[1,5,5-trimethyl-5-[trimethylsilyl)oxy]-3-pentynyl]-7a-methyl-4H-inden-4-on.

g) Einem Gemisch von 0,32 g [3S-(1Z,3α,5β)]-[2-[3,5-Bis[[(1,1-dimethyläthyl)dimethylsilyl]oxy]-2-methylencyclohexyliden]äthyl]diphenylphosphinoxid (J.A.C.S. 104, 1982, 2945) und 8 ml THF werden unter Argon bei -78° 0,32 ml 1,6M n-Butyllithium in Hexan zugesetzt. Die Lösung wird 10 Minuten bei -78° gerührt. Eine Lösung von 0,10 g des Produkts von f) in 2 ml THF wird zugesetzt und die Lösung wird 1,5 Stunden bei -78° gerührt. Dem Gemisch werden 4 ml eines gesättigten wässrigen 1:1 Gemisch von 1M Kaliumnatriumtartrat und 2M Kaliumbicarbonat zugesetzt. Das Gemisch wird auf 25° erwärmt und mit 30 ml einer Lösung von Kaliumnatriumtartrat und 2M Kaliumbicarbonat verdünnt. Die Lösung wird mit Aethylacetat extrahiert. Die vereinigten organischen Phasen werden mit Wasser und dann mit einer Salzlösung gewaschen. Die organische Phase wird getrocknet, filtriert und eingedampft. Das Produkt wird über Silicagel mit 19:1 Hexan-Aethylacetat gereinigt. Man erhält 0,13 g (1α,3β,5Z,7E)-1,3-Bis[[(1,1-dimethyläthyl)dimethylsilyl]-oxy][25-[(trimethylsilyl) oxy]-9,10-secocholesta-5,7,10(19)-trien-23-yn, $[\alpha]^{25}_D$+37,8° (c 0.52, $CHCl_3$).

h) Ein Gemisch von 0,12 g (0,00017 Mol) des Produkt von g) und 8 ml 1% Tetrabutylammoniumfluorid in THF wird 18 Stunden unter Argon gerührt, dann mit Wasser verdünnt und mit Aethylacetat extrahiert. Die vereinigten organischen Phasen werden mit Wasser und einer Salzlösung gewaschen. Die organische Phase wird getrocknet, filtriert und eingedampft. Das Produkt wird über Silicagel mit 2:1 Hexan-Aethylacetat gereinigt. Man erhält 0,048 g (1α,3β,5Z,7E)-9,10-Secocholesta-5,7,10(19)-trien-23-yn-1,3,25-triol, $[\alpha]^{22}_D$+22,8° (c 0.21, $CHCl_3$).

Beispiel 2

a) Ein Gemisch von 2,00 g (6.12 mmol) [1R-[1α(S*),3aβ,4α,7aα]]-β,7a-Dimethyl-4-[[(1,1-dimethyläthyl)dimethylsilyl]oxy]-octahydro-1H-inden-1-äthanol, 2,92 g p-toluolsulfonylchlorid und 50 ml Pyridin wird 19 Stunden unter Argon bei 0° gerührt. Nach Zusatz von Eis wird mit Wasser verdünnt. Das Gemisch wird mit Methylenchlorid extrahiert. Die organische Phase wird mit 1 N $H_2SO_4$, Wasser und gesättigtem wässrigem $NaHCO_3$ gewaschen. Die Lösung wird getrocknet und eingedampft. Der Rückstand wird über Silicagel mit 1:8 Aethylacetat-Hexan chromatographiert. Man erhält 2,81 g (96%) [1R-[1α(S*),3aβ,4α,7aα]]-β,7a-Dimethyl-4-[[(1,1-dimethyläthyl)dimethylsilyl]oxy]-octahydro-1H-iden-1-äthanol-4-methylbenzolsulfonat, $[\alpha]^{25}_D$+34.1° (c 0,92, $CHCl_3$).

b) Einer Lösung von 4.96 ml (Trimethylsilyl)acetylen in 34 ml Dioxan werden bei 5° 22,0 ml 1,6 M Butyllithium in Hexan zugetropft. Nach 30 Min Rühren bei +4° und dann 1,5 Stunden bei 25° wird eine Lösung von 2,81 g des Produkts von a) in 44 ml Dioxan zugetropft. Das Gemisch wird 20 Stunden bei Rückflusstemperatur erhitzt. Bei 0° gehaltenes Salzwasser wird zugesetzt und das Gemisch wird mit Aether extrahiert. Die organische Phase wird mit einer Salzlösung gewaschen, getrocknet und eingedampft. Der Rückstand wird über Silicagel mit Hexan chromatographiert. Man erhält 2,09 g (88%) [1R-[¹α(R*),3aβ,4α,7aα]]-4-[[4-(1,1-Dimethyläthyl)dimethylsilyl]oxy]octahydro-7a-methyl-1H-inden-1-yl]-1-pentynyl]trimethylsilane, $[\alpha]^{25}_D$+46,9° (c 0.95, $CHCl_3$).

c) Einer Lösung von 2,09 g des Produkts von b) in 11 ml Aethanol wird eine Lösung von 2,31 g Silbernitrat in 20 ml 3:1 Aethanol-Wasser zugesetzt. Das Gemisch wird 30 Min. bei 50° gerührt und dann auf 25° abgekühlt. Dann wird eine Lösung von 4.28 g Kaliumcyanid in 11 ml Wasser zugesetzt und das Gemisch wird 2 Stunden bei 25° gerührt. Das Gemisch wird mit Wasser verdünnt und mit Aether etrahiert. Die organische Phase wird mit Wasser gewaschen, getrocknet und eingedampft. Der Rückstand wird über Silicagel mit Hexan chromatographiert. Man erhält 1,63 g (95%) [1R-[1α(R*),3aβ,4α,7aα]]-[[Octahydro-7a-methyl-1-(1-methyl-3-butynyl)-1H-inden-4--yl]oxy]-(1,1-dimethyläthyl)dimethylsilian, $[\alpha]^{25}_D$+53,8° (c0,64, $CHCl_3$).

d) Einer Lösung von 1.20 g des Produkts von c) in 40 ml THF werden 3.70 ml 1.6 M Butyllithium in Hexan bei -75° zugetropft. Nach 30 Min. Rühren bei 75° wird während 10 Min. gasförmiges Hexafluoraceton in das Reaktionsgemisch eingeblasen. Das Gemisch wird 25 Min. bei -75° gerührt. Dann wird ein 1:1 Gemisch von 1 M Kaliumtartrat und 2 M $KHCO_3$ bei 0° zugesetzt. Das Gemisch wird 1 Stunde bei 25° gerührt, dann mit Methylenchlorid extrahiert. Die organische Phase wird mit dem gleichen Salzgemisch gewaschen, getrocknet und eingedampft. Der Rückstand wird über Silicagel mit 5 % Aethylacetat/Hexan chromatographiert. Man erhält

1,78 g (99 %) [1R-[1α(R*),3aβ,4α,7aα]]-[[1,1-Bis(trifluormethyl)-5-[[4-(1,1-dimethyläthyl)-dimethylsilyl]oxy]octahydro]-7a-methyl-1H-inden-1-yl]-2-pentyn-1-ol, $[\alpha]^{25}_D$+34,4° (c 0,42, $CHCl_3$).

e) Einer Lösung von 1,51 g des Produkts von d) in 17 ml Acetonitril und 15 ml THF werden 13,4 ml 48 % HF zugesetzt. Das Gemisch wird 1,5 Stunden bei 25° gerührt, mit Wasser verdünnt und dann mit Methylenchlorid extrahiert. Die organische Phase wird mit gesättigtem wässrigem $NaHCO_3$ gewaschen, getrocknet und eingedampft. Der Rückstand wird über Silicagel mit 1:3 Aethylacetat-Hexan chromatographiert. Man erhält 1,16 g (99 %) [1R-[1α(R*),3aβ,4aα,7aα]]-Octahydro-1-[5-hydroxy-6-trifluor-5-(trifluormethyl)-1-methyl-3-hexynyl]-7a-methyl-1H-inden-4-ol, $[\alpha]^{25}_d$+29,0° (c 0,57, $CHCl_3$).

f) Einer Lösung von 0,200 g (0,518 Mol) des Produkts von e) in 8 ml Methylenchlorid werden 0,304 g Natriumacetat und 0,610 g 2′,2′-Dipyridiniumchlorochromat zugesetzt. Das Gemisch wird 2 Stunden bei 25° gerührt. Dann werden 0,305 g 2′,2′-Dipyridiniumchlorochromat zugesetzt und das Gemisch wird 110 Min. gerührt. Nach Zusatz von 1,1 ml 2-Propanol wird das Gemisch mit Wasser verdünnt und mit 1:1 Aethylacetat-Aether extrahiert. Die organische Phase wird mit Wasser und einer Salzlösung gewaschen, getrocknet und eingedampft. Der Rückstand wird über Silicagel mit 1:1 Aethylacetat-Hexan chromatographiert. Man erhält [1R-[1α(R*),3aβ,7aα]]-Octahydro-1-[5-hydroxy-6-trifluor-5-(trifluormethyl)-1-methyl-3-hexynyl]-7a-methyl-4H-inden-4-on, $[\alpha]^{23}_D$+2,3° (c 0,48, $CHCl_3$).

g) Einer Lösung von 181 ml [3S-(1Z,3α,5β)]-[2-[3,5-Bis[[(1,1-dimethyläthyl)dimethylsilyl]oxy]-2-methylencyclohexyliden]äthyl]diphenylphosphinoxid in 3,5 ml THF werden bei -75° 0,164 ml 1,6 M Butyllithium in Hexan zugesetzt. Nach Rühren wird eine Lösung von 40 mg des Produkts von f) in 2,5 ml THF zugetropft. Das Gemisch wird 110 Min. bei -75° gerührt. Nach Zusatz von einem 1:1 Gemisch von 1 M Kaliumnatriumtartrat und 2 M $KKHCO_3$ wird das Gemisch mit Aethylacetat extrahiert. Die organische Phase wird mit einer Salzlösung gewaschen, getrocknet und eingedampft. Der Rückstand wird über Silicagel mit 1:5 Aethylacetat-Hexan chromatographiert. Man erhält 65 mg (87 %) (1α,3β,5Z,7E)-1,3-Bis[[(1,1-dimethyläthyl)dimethylsilyl]oxy]26,26,26,27,27,2-7-hexafluor-9,10-secocholesta-5,7,10(19)-trien-23-yn-25-ol, $[\alpha]^{23}_D$+38,8°(c 0,17, $CHCl_3$).

h) Einer Lösung von 60 mg des Produkts von g) in 3 ml THF werden 0,58 ml 1 M Tetrabutylammoniumfluorid in THF zugesetzt. Das Gemisch wird 21 Stunden bei 25° gerührt. Nach Zusatz von 2 ml halb gesättigtem wässrigem $NaHCO_3$ wird das Gemisch 15 Min. bei 25° gerührt und dann mit Aethylacetat extrahiert. Die organische Phase wird mit halbgesättigtem wässrigem $NaHCO_3$ und einer Salzlösung gewaschen und dann getrocknet. Die Lösung wird eingedampft und der Rückstand wird über Silicagel chromatographiert. Man erhält 41 mg (98%) 1α,25-Dihydroxy-26,26,26,27,27,27-hexafluor-23-yncholecalciferol, $[\alpha]^{23}_D$+52,0° (c 0,15, MeOH).

Beispiel 3

a) Wie in Beispiel 2g) beschrieben erhält man ausgehend von 0,292 g [S-(Z)]-[2-[5-[[(1,1-Dimethyläthyl)dimethylsilyl]oxy]-2-methylencyclohexyliden]äthyl]diphenylphosphinoxid (J. Org. Chem. 48, 1983, 1416) und 89 mg des Produkts von Beispiel 2f) 118 mg (82%) (3β,5Z,7E)-3-[[(1,1-Dimethyläthyl)dimethylsilyl]oxy]-26,26, 26,27,27,27-hexafluor-9,10-secocholesta-5,7,10(19)-trien-23-yn-25-ol, $[\alpha]^{23}_D$+65,0° (c 0.18, $CHCl_3$).

b) Wie beschrieben in Beispiel 2h) erhält man ausgehend von 0,113 g des Produkts von a) 79 mg (86 %) 25-Hydroxy-26,26,26,27,27,27-hexafluor-23-yncholecalciferol, $[\alpha]^{23}_D$+73,7° (c 0,19, MeOH).

Beispiel 4

a) Wie beschrieben in Beispiel 2g) erhält man ausgehend von 0,243 g [S-(Z)]-[2-[5-[[1,1-Dimethyläthyl)dimethylsilyl]oxy]-2-methylencyclohexyliden]äthyl]diphenylphoshin oxid und 0,112 g des Produkts von Beispiel 1f) 0,174 g (93 %) (3β,5Z,7E)-3-[[(1,1-Dimethylätyl)dimethylsilyl]oxy]-9,10-secocholesta-5,7,10(19)-trien-23-yn-25-yl]oxy]trimethylsilan, $[\alpha]^{23}_D$+79,2° (c, 0,24, $CHCl_3$).

b) Wie beschrieben in Beispiel 2h) erhält man ausgehend von 0,167 g des Produkts a) 0,104 g (94 %) (3β,5Z,7E)-9,10-Secocholesta-5,7,10(19)-trien-23-yn-3,25-diol, $[\alpha]_D$ +98.8° (c 0,16, MeOH).

Im folgenden Beispiel wird die Füllmasse einer Weichgelatinekapsel mit der weiter oben definierten Verbindung C als Wirkstoff angegeben:

| | mg pro Kapsel | |
|---|---|---|
| Verbindung C | 0.00025 | 0.002 |
| Frakt. Kokossnussoel | 199.995 | 199.990 |
| Butyliertes Hydroxyanisol | 0.01 | 0.01 |
| Ascorbylpalmitat | 1.0 | 1.0 |

## Patentansprüche

**Patentansprüche für folgende Vertragsstaaten : AT, BE, CH, DE, FR, GB, IT, LI, LU, NL, SE**

1. Eine Verbindung der Formel

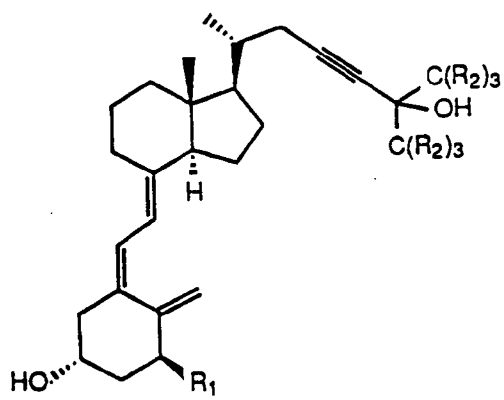

I

worin $R_1$ Wasserstoff oder Hydroxy und $R_2$ Wasserstoff oder Fluor ist.

2. Eine Verbindung gemäss Anspruch 1 aus der Gruppe der folgenden
1α,25-Dihydroxy-23,24-didehydro-26,26,26,27,27,27-hexafluorcholecalciferol,
25-Hydroxy-23,24-didehydro-26,26,26,27,27,27-hexafluorcholecalciferol,
1α,25-Dihydroxy-23,24-didehydrocholecalciferol, und
25-Hydroxy-23,24-didehydrocholecalciferol.

3. Eine Verbindung gemäss Anspruch 1 oder 2 zur Verwendung als therapeutisch aktive Substanz, insbesondere für die Behandlung von Krankheiten, die durch Mangelzustände des Kalzium-Metabolismus charakterisiert sind, speziell von Osteoporose und von renaler Osteodystrophie.

4. Ein Medikament, insbesondere für die Behandlung von Krankheiten, die durch Mangelzustände des Kalzium-Metabolismus charakterisiert sind, speziell von Osteoporose und von renaler Osteodystrophie, enthaltend eine pharmazeutisch effektive Menge einer Verbindung nach Anspruch 1 oder 2, insbesondere in einer Dosierung von etwa 0,1 bis 2 µg, und ein pharmazeutisch geeignetes Trägermaterial.

5. Die Verwendung einer Verbindung nach Anspruch 1 oder 2 für die Herstellung eines Medikaments für die Behandlung von Krankheiten, die durch Mangelzustände des Kalzium-Metabolismus charakterisiert sind, insbesondere der Osteoporose und der renalen Osteodystrophie.

**Patentansprüche für folgenden Vertragsstaat: ES**

1. Ein Verfahren zur Herstellung einer Verbindung

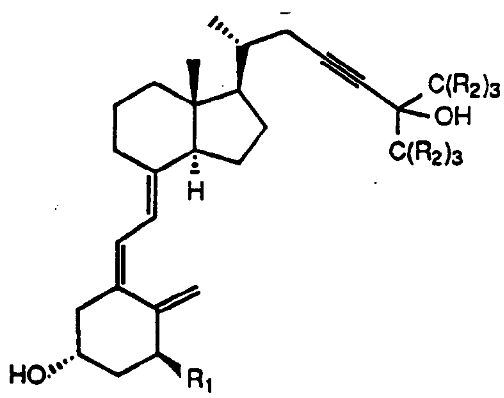

I

worin $R_1$ Wasserstoff oder Hydroxy und $R_2$ Wasserstoff oder Fluor ist, dadurch gekennzeichnet, dass man die Hydroxyschutzgruppen von einer entsprechenden Verbindung, in der die Hydroxygruppen geschützt sind, abspaltet.

2. Ein Verfahren nach Anspruch 1 zur Herstellung einer Verbindung aus der Gruppe der folgenden
1α,25-Dihydroxy-23,24-didehydro-26,26,26,27,27,27-hexafluorcholecalciferol,
25-Hydroxy-23,24-didehydro-26,26,26,27,27,27-hexafluorcholecalciferol
1α,25-Dihydroxy-23,24-didehydrocholecalciferol, und
25-Hydroxy-23,24-didehydrocholecalciferol.

3. Ein Verfahren nach Anspruch 1, daduch gekennzeichnet, dass man die Hydroxyschutzgruppen mittels eines organischen Fluorids in einem Lösungsmittel oder mittels eines $C_{1-8}$-Alkanols oder eines Gemisches von Wasser und einem organischen Lösungsmittel in Gegenwart einer Säure abspaltet.

4. Ein Verfahren zur Herstellung eines pharmazeutischen Präparates, insbesondere für die Behandlung von Krankheiten, die durch Mangelzustände des Kalzium-Metabolismus charakterisiert sind, speziell von Osteoporose und von renaler Osteodystrophie, dadurch gekennzeichnet, dass man eine Verbindung gemäss Anspruch 1 oder 2 in eine galenische Form bringt.

5. Die Verwendung einer Verbindung nach Anspruch 1 oder 2 für die Herstellung eines Medikaments, für die Behandlung von Krankheiten, die durch Mangelzustände des Kalzium-Metabolismus charakterisiert sind, insbesondere der Osteoporose und der renalen Osteodystrophie.

**Patentansprüche für folgenden Vertragsstaat: GR**

1. Ein Verfahren zur Herstellung einer Verbindung

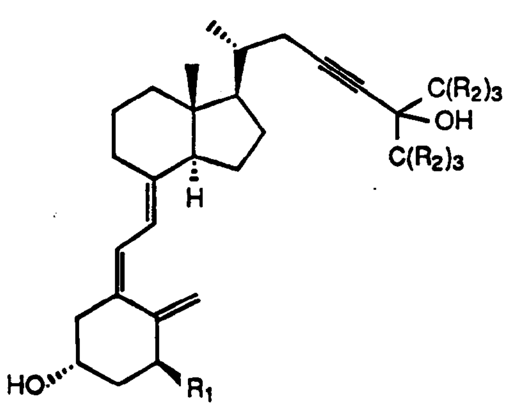

I

worin $R_1$ Wasserstoff oder Hydroxy und $R_2$ Wasserstoff oder Fluor ist, dadurch gekennzeichnet, dass man die Hydroxyschutzgruppen von einer entsprechenden Verbindung, in der die Hydroxygruppen geschützt sind, abspaltet.

2. Ein Verfahren nach Anspruch 1 zur Herstellung einer Verbindung aus der Gruppe der folgenden
1α,25-Dihydroxy-23,24-didehydro-26,26,26,27,27,27-hexafluorocholecalciferol,
25-Hydroxy-23,24-didehydro-26,26,26,27,27,27-hexafluorcholecalciferol

1α,25-Dihydroxy-23,24-didehydrocholecalciferol, und
25-Hydroxy-23,24-didehydrocholecalciferol.

3. Ein Verfahren zur Herstellung eines pharmazeutischen Präparates, insbesondere für die Behandlung von Krankheiten, die durch Mangelzustände des Kalzium-Metabolismus charakterisiert sind, speziell von Osteoporose und von renaler Osteodystrophie, dadurch gekennzeichnet, dass man eine Verbindung gemäss Anspruch 1 oder 2 in eine galenische Form bringt.

4. Die Verwendung einer Verbindung nach Anspruch 1 oder 2 für die Herstellung eines Medikaments, für die Behandlung von Krankheiten, die durch Mangelzustände des Kalzium-Metabolismus charakterisiert sind, insbesondere der Osteoporose und der renalen Osteodystrophie.

## Claims

### Claims for the following Contractings States : AT, BE, CH, DE, FR, GB, IT, LI, LU, NL, SE

1. A compound of the formula

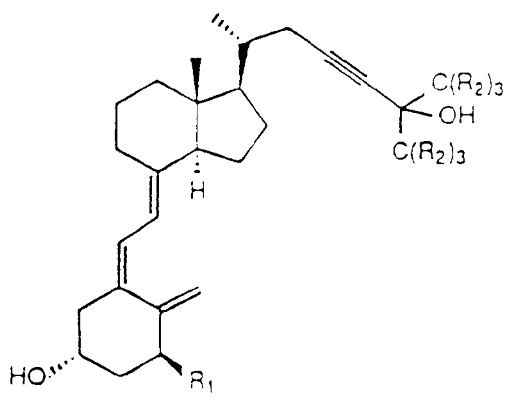

I

wherein $R_1$ is hydrogen or hydroxy and $R_2$ is hydrogen or fluorine.

2. A compound in accordance with claim 1 from the following group:
1α,25-Dihydroxy-23,24-didehydro-26,26,26,27,27,27-hexafluorocholecalciferol,
25-hydroxy-23, 24-didehydro-26, 26, 26, 27, 27, 27-hexafluorocholecalciferol,
1α,25-dihydroxy-23,24-didehydrocholecalciferol, and
25-hydroxy-23,24-didehydrocholecalciferol.

3. A compound in accordance with claim 1 or 2 for the used as a therapeutically active substance, especially for the treatment of diseases which are characterized by metabolic calcium deficiencies, particularly of osteoporosis and of renal osteodystrophy.

4. A medicament, especially for the treatment of diseases which are characterized by metabolic calcium deficiencies, particularly of osteoporosis and renal osteodystrophy, containing a pharmaceutically effective amount of a compound according to claim 1 or 2, especially in a dosage of about 0.1 to 2 µg, and a pharmaceutically suitable carrier material.

5. The use of a compound according to claim 1 or 2 for the manufacture of a medicament for the treatment of diseases which are characterized by metabolic calcium deficiency, especially osteoporosis and renal osteodystrophy.

### Claims for the following Contracting State : ES

1. A process for the preparation of a compound

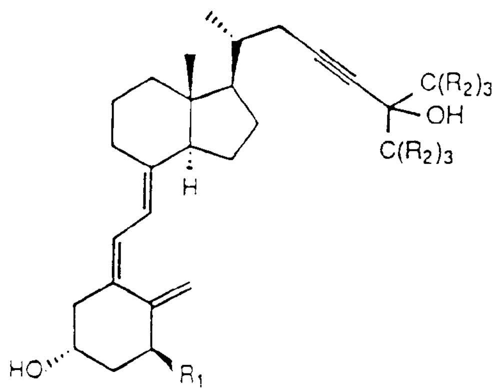

I

wherein $R_1$ is hydrogen or hydroxy and $R_2$ is hydrogen or fluorine,
characterized by cleaving off the hydroxy protecting groups from a corresponding compound in which the hydroxyl groups are protected.

2. A process according to claim 1 for the preparation of a compound from the following group:
1α,25-Dihydroxy-23,24-didehydro-26,26,26,27,27,27-hexafluorocholecalciferol,
25-hydroxy-23, 24-didehydro-26, 26, 26, 27, 27, 27-hexafluorocholecalciferol,
1α,25-dihydroxy-23,24-didehydrocholecalciferol, and
25-hydroxy-23, 24-didehydrocholecalciferol.

3. A process according to claim 1, characterized in that the hydroxy protecting groups are cleaved off using an organic fluoride in a solvent or using a $C_{1-8}$-alkanol or a mixture of water and an organic solvent in the presence of an acid.

4. A process for the manufacture of a pharmaceutical preparation, especially for the treatment of diseases which are characterized by metabolic calcium deficiencies, particularly of osteoporosis and renal osteodystrophy, characterized by bringing a compound as in claim 1 or 2 into a galenical form.

5. The use of a compound according to claim 1 or 2 for the manufacture of a medicament for the treatment of diseases which are characterized by metabolic calcium deficiency, especially osteoporosis and renal osteodystrophy.

**Claims for the following Contracting State : GR**

1. A process for the preparation of a compound

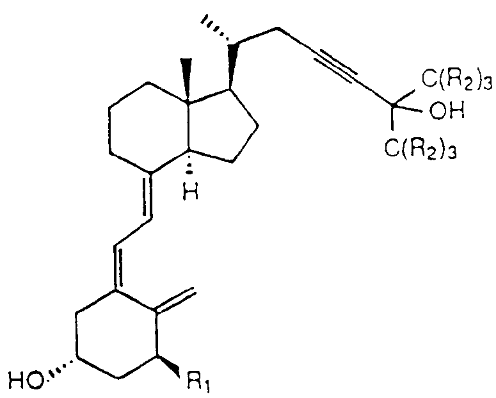

I

wherein $R_1$ is hydrogen or hydroxy and $R_2$ is hydrogen or fluorine,
characterized by cleaving off the hydroxy protecting groups from a corresponding compound in which the hydroxyl groups are protected.

2. A process according to claim 1 for the preparation of a compound from the following group:
1α,25-Dihydroxy-23,24-didehydro-26,26,26,27,27,27-hexafluorocholecalciferol,
25-hydroxy-23, 24-didehydro-26, 26, 26, 27, 27, 27-hexafluorocholecalciferol,
1α,25-dihydroxy-23,24-didehydrocholecalciferol, and 25-hydroxy-23, 24-didehydrocholecalciferol.

3. A process for the manufacture of a pharmaceutical preparation, especially for the treatment of diseases which are characterized by metabolic calcium deficiencies, particularly of osteoporosis and renal osteodystrophy, characterized by bringing a compound as in claim 1 or 2 into a galenical form.

4. The use of a compound according to claim 1 or 2 for the manufacture of a medicament for the treatment of diseases which are characterized by metabolic calcium deficiency, especially osteoporosis and renal osteodystrophy.

## Revendications

### Revendications pour les Etats contractants suivants : AT, BE, CH, DE, FR, GB, IT, LI, LU, NL, SE

1. Composé de formule

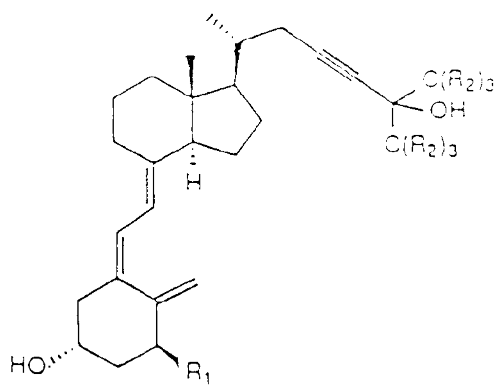

I

dans laquelle $R_1$ est un hydrogène ou un hydroxy et $R_2$ est un hydrogène ou un fluor.

2. Composé selon la revendication 1 du groupe des suivants
1α,25-Dihydroxy-23,24-didéhydro-26,26,26,27,27,27-hexafluorocholécalciférol,
25-Hydroxy-23,24-didéhydro-26,26,26,27,27,27-hexafluorocholécalciférol,
1α-25-Dihydroxy-23,24-didéhydrocholécalciferol, et
25-Hydroxy-23,24-didéhydrocholécalciférol.

3. Composé selon la revendication 1 ou 2 aux fins d'application comme substance thérapeutiquement active, en particulier pour le traitement des maladies qui se caractérisent par des états de déficience du métabolisme du calcium, en particulier de l'ostéoporose et de l'ostéodystrophie rénale.

4. Médicament, en particulier pour le traitement des maladies qui sont caractérisées par des états de déficience du métabolisme du calcium, en particulier de l'ostéoporose et de l'ostéodystrophie rénale, contenant une quantité pharmaceutiquement efficace d'un composé selon la revendication 1 ou 2, en particulier en une dose d'environ 0,1 à 2 μg, et un support pharmaceutiquement approprié.

5. Application d'un composé selon la revendication 1 ou 2 à la préparation d'un médicament pour le traitement des maladies qui sont caractérisées par des états de déficience du métabolisme du calcium, en particulier de l'ostéoporose et de l'ostéodystrophie rénale.

### Revendications pour l' Etat contractant suivant : ES

1. Procédé de préparation d'un composé

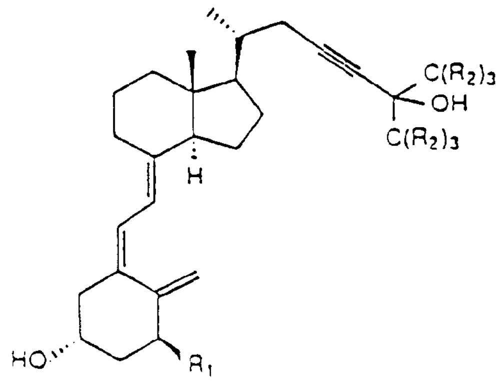

I

où $R_1$ est un hydrogène ou un hydroxy et $R_2$ est un hydrogène ou un fluor,
caractérisé en ce qu'on sépare les groupes protecteurs d'hydroxy d'un composé correspondant dans lesquels les groupes hydroxy sont protégés.

2. Procédé selon la revendication 1 de préparation d'un composé du groupe des suivants
1α,25-Dihydroxy-23,24-didéhydro-26,26,26,27,27,27-hexafluorocholécalciférol,
25-Hydroxy-23,24-didéhydro-26,26,26,27,27,27-hexafluorocholécalciférol
1α-25-Dihydroxy-23,24-didéhydrocholécalciferol, et
25-Hydroxy-23,24-didéhydrocholécalciférol.

3. Procédé selon la revendication 1, caractérisé en ce qu'on sépare les groupes protecteurs d'hydroxy au moyen d'un fluorure organique dans un solvant ou au moyen d'un alcanol en $C_1$ à $C_8$ ou d'un mélange d'eau et d'un solvant organique en présence d'un acide.

4. Procédé de préparation d'une préparation pharmaceutique, en particulier pour le traitement des maladies qui sont caractérisées par les états de déficience du métabolisme du calcium, en particulier de l'ostéoporose et de l'ostéodystrophie rénale, caractérisé en ce qu'on met un composé selon la revendication 1 ou 2 sous une forme galénique.

5. Application d'un composé selon la revendication 1 ou 2 à la préparation d'un médicament pour le traitement des maladies qui sont caractérisées par des états de déficience du métabolisme du calcium, en particulier de l'ostéoporose et de l'ostéodystrophie rénale.

**Revendications pour l' Etat contractant suivant : GR**

1. Procédé de préparation d'un composé

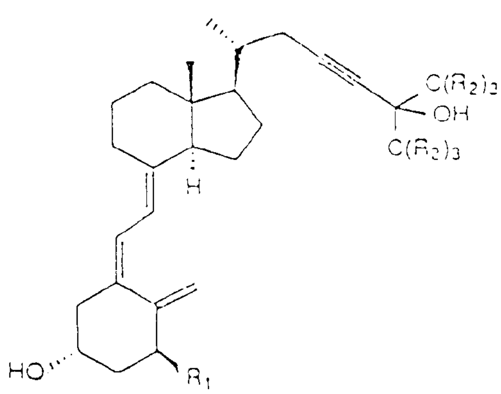

:

où $R_1$ est un hydrogène ou un hydroxy et $R_2$ est un hydrogène ou un fluor,
caractérisé en ce qu'on sépare les groupes protecteurs d'hydroxy d'un composé correspondant dans lequel les groupes hydroxy sont protégés.

2. Procédé selon la revendication 1 de préparation d'un composé du groupe des suivants
1α,25-Dihydroxy-23,24-didéhydro-26,26,26,27,27,27-hexafluorocholécalciférol,
25-Hydroxy-23,24-didéhydro-26,26,26,27,27,27-hexafluorocholécalciférol,
1α-25-Dihydroxy-23,24-didéhydrocholécalciferol, et 25-Hydroxy-23,24-didéhydrocholécalciférol.

3. Procédé de préparation d'une préparation pharmaceutique, en particulier pour le traitement des maladies qui sont caractérisées par des états de déficience du métabolisme du calcium, en particulier de l'ostéoporose et de l'ostéodystrophie rénale, caractérisé en ce qu'on met sous une forme galénique un composé selon la revendication 1 ou 2.

4. Application d'un composé selon la revendication 1 ou 2 à la préparation d'un médicament pour le traitement des maladies qui sont caractérisées par des états de déficience du métabolisme du calcium, en particulier de l'ostéoporose et de l'ostéodystrophie rénale.